# EUROPEAN PATENT APPLICATION

(11) **EP 4 417 152 A1**
(43) Date of publication of application: **21.08.2024**
(21) Application number: 23156980.7
(22) Date of filing: 16.02.2023
(51) Int. Cl.: A61B 18/14, A61B 5/02, A61M 5/00

(54) **CATHETER AND METHOD FOR CARDIAC SURGERY GENERATING AN INTERATRIAL SHUNT**

(71) Applicant: BIOTRONIK SE & Co. KG, 12359 Berlin (DE)
(72) Inventor: Wegerich, Franziska, 12055 Berlin (DE); Ebert, Henning, 12437 Berlin (DE); Holzinger, Steffen, 12437 Berlin (DE)
(74) Representative: Biotronik Corporate Services SE

(57) **Abstract**

Catheter (1) and a method for cardiac surgery are described. The catheter comprises an elongate catheter tip (3) and a deformable electrode (5) arranged at the catheter tip. The deformable electrode is configured for applying electrical RF energy to tissue for locally ablating the tissue. The deformable electrode is configured to be selectively deformed between a retracted configuration (7) and an expanded configuration (9), wherein, in the expanded configuration, the deformable electrode extends along a larger cross-sectional area (11) than in the retracted configuration, the cross-sectional area extending perpendicular with regards to a longitudinal direction of the catheter tip. A maximum extension of the cross-sectional area of the deformable electrode in the expanded configuration is smaller than 9 mm. In a cardiac surgery, the catheter tip may be transferred through a septum (49) into a left atrium (47) of a heart. The deformable electrode may then be expanded and RF energy may be applied upon displacing the expanded electrode relative to the septum, thereby ablating tissue from the septum and forming a wide opening (50") serving as an interatrial shunt. Optionally, a pressure sensor (13) is provided at the catheter tip, thereby allowing controlling a success of the cardiac surgery.

## Description

The present invention relates to a catheter and a method for cardiac surgery.

About one million new patients per year in the USA suffer from heart failure. This disease has a high mortality and many of the patients suffer from deficiencies such as distress or limited physical capabilities. There are different types of heart failure and various different reasons for an occurrence of a heart failure. However, it is common to most heart failure types that the pressure in the left atrium, i.e. left atrial pressure (LAP), increases. Such LAP typically represents a measure indicating a severity of the heart failure. In cases where the pressure may not be reduced using for example a heart failure medicine, a back pressure in the lungs and/or accumulation of water in the lungs may occur.

For patients where the left atrial pressure may not successfully be controlled using medication, alternative approaches using cardiac surgery are currently developed. For example, a shunt may be generated between the left atrium and the right atrium in order to enable a partial equilibration of pressures between both atria, thereby possibly relieving a patient's medical condition or discomfort. For such interatrial shunting in a heart, an opening between the right and left atria is generated. Thereby, excess volume in the left atrium may be shunted to the right atrium, the left atrial pressure may be reduced and a pulmonary artery pressure may be decreased. As a result, inter alia, pulmonary congestion and heart failure events may be reduced, and a functional status may be improved and symptoms may be relieved for the patient.

Several approaches for generating atrial shunts are currently developed, part of them presently being under a clinical evaluation. For example, in one approach, an orifice plate device is implanted into a septum dividing the left and right atria, the medical device being permanently fixed to the septum and keeping a hole open in the septum, the hole having been generated in a surgery procedure, in particular a transcatheter procedure. In another approach, an opening is punched into the septum. In a further approach, the septum is first punctured and an initial opening is then widened and fixed using RF ablation with a selfexpanding stent.

US 2020/0261704 A1, WO 2020/094085 A1, WO 2020/094087 A1, WO 2020/094094 A1 and WO 2020/259492 A1 describe some conventional approaches.

The conventional approaches may suffer from various deficiencies such as resulting in medical problems for the patient, requiring complex surgery procedures, insufficient adaptability to individual physiologies of different patients, etc.

It may therefore be an object of the present invention to provide a device and a method allowing improved cardiac surgery for reducing negative impacts of a heart failure. Particularly, it may be an object of the present invention to provide an approach for fast, effective and safe generation of an interatrial shunt in a heart.

Such objects may be met with the subject-matter of the independent claims. Advantageous embodiments are defined in the dependent claims as well as the corresponding specification and figures.

According to a first aspect of the present invention, a catheter for cardiac surgery is presented, the catheter comprising an elongate catheter tip and a deformable electrode arranged at the catheter tip. The deformable electrode is configured for applying electrical RF energy to tissue for locally ablating the tissue. The deformable electrode is configured to be selectively deformed between a retracted configuration and an expanded configuration. Therein, in the expanded configuration, the deformable electrode extends along a larger cross-sectional area than in the retracted configuration, the cross-sectional area extending perpendicular with regards to a longitudinal direction of the catheter tip. Furthermore, a maximum extension of the cross-sectional area of the deformable electrode in the expanded configuration is smaller than 9mm.

According to a second aspect of the invention, a method for cardiac surgery is presented. Therein, a catheter comprising an elongate catheter tip and a deformable electrode arranged at the catheter tip is provided, wherein the deformable electrode is configured for applying electrical RF energy to tissue for locally ablating the tissue, and wherein the deformable electrode is configured to be selectively deformed between a retracted configuration and an expanded configuration, wherein, in the expanded configuration, the deformable electrode extends along a larger cross-sectional than in the retracted configuration, the cross-sectional area extending perpendicular with regards to a longitudinal direction of the catheter tip. The method furthermore comprises at least the following step, preferably in the indicated order:
- introducing the catheter with its catheter tip into a right atrium of the heart;
- optionally puncturing a septum separating the right atrium from a left atrium of the heart thereby generating an opening in the septum; and
- transferring the catheter tip, with the deformable electrode being in the retracted configuration, through the generated opening or an already existing opening into the left atrium such that at least a portion of the deformable electrode is positioned within the left atrium,
- deforming the deformable electrode into its expanded configuration; and
- displacing the catheter tip such that the deformable electrode in its expanded configuration contacts a portion of the septum while applying electrical RF energy via the deformable electrode to tissue of the septum, thereby ablating tissue of the septum for widening the opening.

Within the frame of the application the direction along the catheter axis (the axis of the catheter tip) is to be understood as longitudinal direction of the catheter (tip). The direction along an axis perpendicular to the catheter axis is to be understood as radial direction.

Within the frame of the application deformable electrode may be understood as an electrode which can be selectively and continuously deformed between a retracted and an expanded configuration. An electrode made of a shape memory material would be either in its memory state (e.g. the expanded state) where no external forces act on the electrode or in its forced state (e.g. the retracted state) where the shape is maintained by the applied external forces. In contrast to such an electrode the deformable electrode may be deformed in any shape (e.g. any shape between the expanded and retracted state) and maintain the shape even in the absence of an external force. A deformable electrode within the frame of the application may change via plastic deformation between retracted configuration and an expanded configuration and vice versa.

Ideas underlying embodiments of the present invention may be interpreted as being based, inter alia, on the following observations and recognitions.

Briefly summarised in a non-limiting manner, embodiments of the present invention relate to a catheter and a method adapted for a specific type of cardiac surgery. Therein, a deformable electrode is arranged at a catheter tip, such deformable electrode being specifically and reversibly expandable. During cardiac surgery, a septum between the left and right atria may then first be punctured before then transferring the catheter tip through an opening generated thereby. The opening may be generated by puncturing the septum using the catheter or by using a transseptal needle. Upon having been introduced into the left atrium, the electrode may be deformed into its expanded configuration, thereby enlarging its cross-sectional area. The electrode is expanded in the radial direction. The catheter tip is then displaced such that its expanded electrode comes into contact with a portion of the septum. Meanwhile, using the expanded electrode, RF energy is applied to tissue of the septum, thereby ablating at least part of this tissue in order to permanently widen the opening through the septum for generating an effective interatrial shunt.

As an optional additional feature, the catheter may comprise a pressure sensor. Such pressure sensor enables measuring a blood pressure for example within the right atrium and/or within the left atrium before and/or after puncturing the septum, wherein a value of the measured blood pressure may be beneficially used for adapting the cardiac surgery procedure. For example, a diameter of the permanently widened opening generated by RF ablation using the expanded electrode and/or subsequent processing steps during the cardiac surgery procedure may be determined or planned based on the acquired blood pressure value(s).

In the following, characteristics of embodiments of the present invention will be described in more detail.

First, possible features, characteristics and/or advantages of the catheter according to embodiments of the first aspect of the invention will be described.

The catheter typically comprises an elongate shaft with a handle at a proximal end of the shaft and an elongate catheter tip at a distal end of the shaft. In a surgical procedure, the catheter may be introduced into blood vessels, e.g. the femoral vein, of a patient. The shaft may be deflected using suitable mechanisms at the handle, thereby allowing steering and guiding the shaft through the vessels until reaching a heart of the patient. Such shaft typically has a length of several tens of centimetres and a diameter of typically less than 8 mm, in most cases less than 6 mm, in particular less than 5mm. Generally, the shaft includes elongate lumens through which for example steering wires for deflecting the shaft may extend, cables for supplying electric energy to devices or structures at the catheter tip may be arranged and/or fluids such as contrast fluids, cooling fluids, etc. may be supplied.

At the distal catheter tip, a deformable electrode is provided. The deformable electrode consists of or comprises electrically conductive material such as e.g. metal. Such electrode may be supplied with electric energy. The energy may be supplied as RF energy (RF = radio frequency). During a surgery procedure, one or more further electrodes may be arranged at a body of a patient, e.g. somewhere at a back or thigh of the patient. Accordingly, the RF energy applied to the electrode at the catheter tip may result in electric RF currents being induced between this catheter electrode and the further electrode(s). Such induced currents may be generated such that tissue being in direct contact with the electrode at the catheter tip is locally heated and thereby ablated, i.e. permanently removed and/or cicatrised.

The electrode at the catheter tip is reversibly deformable between a first configuration, referred to herein as retracted configuration, and a second configuration, referred to herein as expanded configuration. Therein, the retracted configuration and the expanded configuration differ with respect to their cross-sectional areas, such cross-sectional areas being measured in a plane extending perpendicular to a longitudinal direction of the catheter tip (e.g. measured in the radial direction). For example, in the retracted configuration, the deformable electrode may extend along a relatively small cross-sectional area corresponding approximately to a cross-sectional area of the shaft of the catheter, i.e. protruding at most slightly beyond the catheter tip in a radial direction. In other words, in the retracted configuration, the deformable electrode preferably is substantially flush with regards to the catheter tip. In contrast hereto, in the expanded configuration, the deformable electrode may extend along a substantially larger cross-sectional area, such cross-sectional area typically being substantially larger than the cross-sectional area of the shaft of the catheter. Accordingly, in the expanded configuration, the deformable electrode generally substantially protrudes from the catheter tip in the radial direction.

The deformable electrode may have a ring-like configuration. Particularly, the deformable electrode may have a tube-like shape. Accordingly, the deformable electrode may extend along a circumference of the catheter tip.

The deformable electrode is configured such that a maximum extension of its cross-sectional area in the expanded configuration is smaller than 9 mm or smaller than 8mm, smaller than 7mm or smaller than 6mm. In other words, the deformable electrode may, due to its physical and/or functional implementation, be configured such that its cross-sectional area may not be expanded beyond a predetermined maximum value of 9 mm or less. Accordingly, even upon being in its fully extended expanded configuration, the deformable electrode still has a relatively small cross-sectional area or diameter. Therein, such maximum cross-sectional area roughly corresponds to a cross-sectional area of the opening in the septum to be formed by the cardiac surgery as an interatrial shunt.

Generally, the maximum cross-sectional area of the deformable electrode in its expanded configuration is located somewhere at or close to a centre of the longitudinal extension of the deformable electrode. In other words, the expanded electrode generally has its maximum cross-sectional area not at its distal-most or proximal-most ends but somewhere in between such ends. Accordingly, the cross-sectional area of the expanded electrode increases from both of its longitudinal ends towards a centre of the electrode. Accordingly, the expanded electrode generally has a flank directed to a proximal end of the catheter and another flank directed to a distal end of the catheter.

According to an embodiment, the catheter further comprises a pressure sensor arranged at the catheter tip, the pressure sensor being configured for sensing a blood pressure within a cardiac chamber.

The pressure sensor may for example be a micro-electronical and/or micro-mechanical device. The pressure sensor may be small enough to be arranged at the catheter tip. The pressure sensor may be exposed at the catheter tip such as to enable direct contact with fluid surrounding the catheter tip. Particularly, the pressure sensor may be configured for sensing pressures typically occurring in a cardiac chamber. The pressure sensor may be connected to a controller circuitry or evaluation circuitry via wires extending along the shaft of the catheter.

As explained in more detail further below with regards to the method for cardiac surgery, the pressure sensor may be used during a surgical procedure for beneficially adapting such procedure to measured conditions within the heart, such conditions correlating with the blood pressure within one or more cardiac chamber.

According to a further specified embodiment, the pressure sensor may be arranged distally with regards to the deformable electrode.

In such distal arrangement, the pressure sensor is positioned between the deformable electrode and a distal catheter tip end. Accordingly, the pressure sensor may measure blood pressures in a volume which is located external to and distal from the deformable electrode. Such option of measuring blood pressures distal from the deformable electrode may be beneficial upon using the catheter in a cardiac surgical procedure, as described in more detail below.

It is to be noted that, alternatively, the pressure sensor may be arranged proximal with regards to the deformable electrode. As a further alternative, multiple pressure sensors may be provided at the catheter tip, wherein one or more pressure sensors may be arranged at a distal location and/or one or more pressure sensors may be arranged at a proximal location relative to the deformable electrode.

According to an embodiment, the deformable electrode may be configured for applying the electrical RF energy at an outermost area of the electrode being in the expanded configuration.

In other words, due to its physical and/or functional implementation, the deformable electrode may be adapted such that it may contact adjacent tissue with an outermost area of the electrode while applying electrical RF energy at this outermost area such that the tissue is ablated in a region contacting this outermost area. Expressed differently, the RF energy may be applied along a maximum circumference of the deformable electrode in its expanded configuration. The outermost area of the electrode may be positioned at or near the centre of the deformable electrode along its longitudinal direction. Accordingly, for example a through-hole extending through the cardiac septum may be generated and/or widened due to ablation induced by the RF energy applied by the expanded electrode. Such through-hole may then have a diameter roughly corresponding to a diameter of the outermost area of the expanded deformable electrode.

Optionally, electrical RF energy may be supplied along the entire extension of the deformable electrode such that the RF energy may be applied to tissue independent of whether the tissue contacts the deformable electrode at its outermost area, i.e. at an area of maximum cross-sectional extension, or at any position at which the deformable electrode has a smaller cross-sectional area, i.e. at a flank of the deformable electrode.

As a further option, the deformable electrode may be provided with two or more sections, wherein electrical RF energy may be applied selectively via one of these sections while the other section(s) may be temporarily inactive. For example, in a specific situation during a cardiac surgery, it may be preferable to supply electrical RF energy via the distal flank of the expanded electrode whereas in another situation during the cardiac surgery, it may be preferable to supply the RF energy via the proximal flank of the expanded electrode, while the respective other flank of the expanded electrode being inactive and therefore not applying heat to the surrounding tissue or blood. Such option of enabling transmitting RF energy only via a specific section of the deformable electrode while another section remaining inactive may be used to control local heat generation during a surgical procedure. For example, it may be avoided that heat is generated in an area where the expanded electrode is not in direct contact with tissue of for example the septum but is only in contact with blood surrounding the septum. Due to such controllable heat generation, thrombogenesis and/or other negative medical effects may be avoided or minimised.

According to an embodiment, the deformable electrode may comprise an inflatable balloon and an electrically conductive structure at an outer surface of the balloon, the conductive structure being deformable together with the balloon upon the balloon being inflated or deflated.

In other words, the deformable electrode may be implemented with a balloon which may be controllably expanded by injecting a fluid into the balloon thereby inflating the balloon and which may be controllably retracted by draining the fluid from the balloon thereby deflating the balloon. The deformable electrode furthermore comprises a conductive structure which is supported by the balloon, held by the balloon and/or fixed to the balloon in a manner such that the conductive structure deforms together with the balloon upon inflation or deflation. Accordingly, the conductive structure may be used for applying the electrical RF energy and may be switched from the retracted configuration to the expanded configuration of the deformable electrode, and vice versa, in a controllable manner by injecting fluid or draining fluid from the balloon. Thereby, the cross-sectional area of the deformable electrode and its conductive structure may be controlled and adjusted very precisely. The fluid may be injected or drained via specific lumens extending through the catheter shaft and ending at openings at the catheter tip discharging into the balloon.

According to a further specified embodiment, the electrically conductive structure may comprise a wire mesh comprising electrically conductive wire portions extending along a circumference of the balloon. Alternatively, the electrically conductive structure may comprise an electrically conductive layer structure applied on an outer surface of the balloon.

In the first alternative, the mesh may comprise a single wire or a multitude of wires. The wires may be exposed, i.e. may not comprise any sheath or surrounding isolation. The wire(s) may extend along a longitudinal extension of the deformable electrode while surrounding the circumference of the balloon. The mesh may be woven or non-woven. The mesh and its wire(s) may be elastically flexible. Accordingly, upon the balloon being inflated, the mesh may be expanded together with the balloon. When the balloon is deflated, the mesh may retract together with the balloon. Accordingly, the electrically conductive mesh may easily and reversibly be expanded and retracted.

In the second alternative, the electrically conductive layer structure may be applied directly onto the outer surface of the balloon. For example, one or preferably a multitude of electrically conductive lines may be attached to the outer surface of the balloon. The lines may be arranged in a distributed and/or meander-type pattern. In other words, the electrically conductive structure does preferably not cover the entire outer surface of the balloon as a continuous layer but the lines forming the electrically conductive structure cover only portions of the outer surface of the balloon, wherein neighbouring lines are spaced from each other. Accordingly, the balloon may be easily inflated or deflated thereby being expanded and retracted, respectively, while the electrically conductive structure at its outer surface may also expand or retract accordingly, without risking cracking or breaking the electrically conductive structure due to mechanical stress. For example, the electrically conductive structure may be applied to the outer surface of the balloon by printing techniques such as screen printing, roller printing, inkjet printing, etc. using an electrically conductive paste or ink. Alternatively, the electrically conductive structure may be applied to the outer surface using deposition techniques such as PVD (physical vapor deposition) or CVD (chemical vapor deposition), possibly including a use of suitable masks.

According to an embodiment, the catheter tip may comprise a first tip portion and a second tip portion being longitudinally displaceable with respect to the first tip portion. Therein, the deformable electrode may comprise a proximal end portion, a middle portion and a distal end portion, wherein the proximal end portion is fixed at the first tip portion and the distal end portion is fixed at the second tip portion.

With such configuration of the catheter tip, the first tip portion may be controllably displaced longitudinally relative to the second tip portion during a surgery procedure, thereby also displacing the proximal end portion and the distal end portion of the deformable electrode longitudinally with respect to each other. Accordingly, the middle portion of the deformable electrode may be displaced between a retracted configuration and an expanded configuration. In the retracted configuration, the middle portion is stretched between the proximal end portion and the distal end portion upon these end portions being arranged at a larger longitudinal distance from each other. In the expanded configuration, the middle portion is bulged between the proximal end portion and the distal end portion upon these end portions being arranged at a smaller longitudinal distance from each other. Accordingly, by displacing the first and second portions longitudinally relative to each other, the deformable electrode may be selectively stretched at its middle portion to the retracted configuration or bulged at its middle portion to the expanded configuration. Thereby, the cross-sectional area of the deformable electrode may be adapted easily and precisely.

According to an embodiment, the catheter comprises a first deformable electrode portion and a second deformable electrode portion. Therein, each deformable electrode portion is configured to be selectively and individually deformed between a retracted configuration and an expanded configuration, wherein, in the expanded configuration, the electrode portion extends along a larger cross-sectional area than in the retracted configuration, the cross-sectional area extending perpendicular with regards to a longitudinal direction of the catheter tip. The first deformable electrode portion is arranged at the catheter tip proximally and spaced apart with regards to the second deformable electrode portion. For at least one of the deformable electrode portions, a maximum extension of the cross-sectional area of the electrode in the expanded configuration is smaller than 9mm.

In other words, the catheter may comprise two separate deformable electrode portions arranged at different longitudinal positions along the catheter tip. The electrode portions may be spaced apart from each other in the longitudinal direction of the catheter. A spacing may be for example between 0.2 mm and 10 mm, preferably between 0.5 mm and 3 mm, preferably between 0.5mm and 1mm. Each electrode portion may be deformed individually, i.e. independent from a configuration of the other electrode portion. Both electrode portions may be deformed from a retracted configuration to an expanded configuration. The retracted configuration and the expanded configuration may be identical or different for the first electrode portion as compared to the second electrode portion. This means that for example in their expanded configuration, the first and second electrode portions may have identical or different cross-sectional areas. Also the deformation mechanism for the first and second deformable electrode portions may be same or different. For example, one of the electrode portions may be deformable using a balloon whereas the other electrode portion may be deformable in its bulging middle portion by longitudinally displacing a first catheter tip portion relative to a second catheter tip portion with proximal and distal end portions of the deformable electrode portion being fixed to the first and second catheter tip portions. At least one of the deformable electrodes is configured such that its cross-sectional area in the expanded configuration extends to a maximum of at most 9 mm, in particular less then 8mm, in particular less than 7mm or less than 6mm.

Having two separately deformable electrode portions, the catheter tip may be easily and stably temporarily fixed for example at the septum. Therein, the catheter tip may be arranged such that one of the deformable electrode portions is arranged distally to the septum whereas the other one is arranged proximal to the septum, thereby interposing the septum between the distal and proximal deformable electrode portions such that the catheter tip may be temporarily held and supported by the septum.

According to an embodiment, a distal end of the catheter tip may be configured for puncturing a cardiac septum.

For example, the distal end of the catheter tip may be pointed or sharp such that, using the catheter tip, the cardiac septum may be penetrated and punctured. Accordingly, the catheter itself with its pointed or sharp catheter tip may be used for generating a small hole through the cardiac septum through which a more proximal portion of the catheter tip may then be transferred until the retracted deformable electrode reaches a volume at an opposite side of the cardiac septum.

However, it has to be noted that, instead of using the catheter tip itself, the cardiac septum may be punctured with a separate device such as for example a separate transseptal needle in order to prepare the first small opening therein.

According to an embodiment, the catheter comprises a further electrode arranged at a distal end of the catheter tip. Such further electrode may be configured for applying electrical RF energy to tissue for locally ablating the tissue. Alternatively or additionally, such further electrode may be configured for sensing an intracardiac electrogram.

In the first alternative, the distal end of the catheter tip forms the further electrode via which RF energy may be transmitted to adjacent tissue in order to thereby locally ablate the tissue. For example, such ablating functionality at the catheter tip end may be used upon penetrating and puncturing the cardiac septum. Therein, instead of or additionally to being pointed or sharp, the catheter tip end may ablate tissue of the septum, thereby supporting the generation of a small hole through the septum.

In the second alternative, the further electrode may be used for sensing an intracardiac electrogram (IEGM). The information about the IEGM may be used during the cardiac surgery procedure for example for orientation purposes.

The further electrode may be connected via wires extending through the catheter shaft to an energy supply and/or a controller for providing RF energy and/or for processing the IEGM.

According to an embodiment, the catheter may further comprise a contrast fluid channel for supplying a contrast fluid and ejecting the contrast fluid at a channel opening at the catheter tip.

In other words, one or more channel openings may be provided at the catheter tip and may be connected to a channel referred to herein as contrast fluid channel extending along a lumen in the catheter shaft. Proximally, the contrast fluid channel may be connected to a contrast fluid reservoir. Accordingly, a contrast fluid may be supplied through the channel and injected from the channel opening into a volume adj acent to the catheter tip. The contrast fluid may be a fluid which locally increases a contrast for example in an x-ray image. Locally injecting contrast fluid may support orienting the catheter tip during a surgical procedure. The channel opening(s) may be arranged at a proximal position and/or at a distal position relative to the deformable electrode at the catheter tip.

Additionally, for more contrast and/or visibility in an x-ray image acquired during the cardiac surgery procedure, one or more x-ray markers may be provided at the catheter tip. Such x-ray markers may comprise a material which substantially absorbs or reflects x-rays, such that the x-ray marker generates a high contrast in an x-ray image. The x-ray marker(s) may be arranged at a proximal position and/or at a distal position relative to the deformable electrode at the catheter tip.

According to an embodiment, the catheter may further comprise a cooling fluid channel for supplying a cooling fluid (e.g. isotonic saline solution) to at least a portion of the deformable electrode.

The cooling fluid channel may be implemented by another lumen extending through the catheter shaft and may end at one or more channel openings at the catheter tip. Proximally, the cooling fluid channel may be connected to a cooling fluid reservoir and an optional pump to provide a defined flow from the fluid reservoir to the openings at the catheter tip. Accordingly, cooling fluid may be supplied along the cooling fluid channel and may be ejected at a channel opening for locally cooling a portion of the deformable electrode. Instead of ejecting the cooling fluid at the channel opening, the cooling fluid channel may be established as a closed-loop system such that cooling fluid may be transferred to a portion of the deformable electrode and may then be further guided back to the cooling fluid reservoir. The portion of the deformable electrode being cooled may be for example an area of the electrode which is not in direct contact with the septum during active RF energy supply. Thereby, such portion distant from the septum may be actively cooled with the cooling fluid, thereby, inter alia, possibly reducing a risk of generating a thrombus.

Next, possible features, characteristics and/or advantages of the method according to embodiments of the second aspect of the invention will be described.

In the proposed method for cardiac surgery, a catheter is provided. The catheter is introduced into blood vessels in a patient's body. The catheter has the characteristics as defined further above for the second aspect of the invention. For example, the catheter may be embodied as described further above for embodiments of the first aspect of the invention.

First, the catheter is transferred through the patient's body until being introduced with its catheter tip in the right atrium of the heart. Subsequently, the septum separating the right and left atria is punctured, Thereby, a small opening is generated in the septum. Alternatively there may be already an opening in the septum generated by the previous use of a transseptal needle. Through this opening, the catheter tip is transferred until at least a portion of the deformable electrode is positioned within the neighbouring left atrium. During this transferral, the deformable electrode is in its retracted configuration such as to be easily pushed through the opening. When having reached the intended position in the left atrium, the deformable electrode is deformed into its expanded configuration. Then, the catheter tip is displaced such that its expanded deformable electrode comes into mechanical contact to a portion of the surface of the septum. When being in such contact, electrical RF energy is applied to the tissue of the septum via the expanded deformable electrode. Thereby, tissue of the septum is ablated and the opening is widened to a cross-sectional area roughly corresponding to the cross-sectional area of the expanded deformable electrode. The widened opening then forms an interatrial shunt between both atria.

According to an embodiment, the method further comprises measuring a pressure within the right atrium and, optionally, determining a degree of expansion into which the deformable electrode is deformed based on the measured pressure in the right atrium.

In other words, preferably prior to puncturing the septum, a pressure sensor provided at the catheter tip is used for measuring the blood pressure within the right atrium. This right atrial pressure (RAP) may be used for controlling a success of the cardiac surgery. For example, the RAP measured before puncturing the septum may later be compared to a RAP measured after preparing the interatrial shunt. Both measured RAP values and particularly a comparison of these values may provide valuable information about an existing heart failure and/or about a success of the cardiac surgery for relieving such heart failure.

According to an embodiment, the method further comprises measuring a pressure within the left atrium and, optionally, determining subsequent processing steps based on the measured pressure in the left atrium.

The left atrial pressure (LAP) may be measured additionally to measuring the RAP. Both pressure values individually and particularly a ratio of both pressure values may provide valuable information about an existing heart failure and/or about a success of the cardiac surgery. The LAP may be measured after the catheter tip has been transferred to the left atrium, using the same pressure sensor provided at the catheter tip as used for a preceding measurement of the RAP. Alternatively, the catheter tip may be provided with two separate pressure sensors for measuring each of the RAP and the LAP.

For example, based on the information about the actual left atrial pressures, in particular based on the difference between right atrial pressure and left atrial pressure, an intended size or cross-sectional area of an opening of the interatrial shunt to be produced may be determined for example by calculation or based on prior experiences. Taking into account this determination, the deformable electrode may then be expanded to acquire a cross-sectional area which at least roughly corresponds to the intended size or cross-sectional area of the opening for the interatrial shunt. Accordingly, by measuring the LAP, medical results of a cardiac surgery may be improved.

According to an additional embodiment, the method comprises, subsequent to the step of displacing the catheter tip for widening the opening in the septum, expanding the deformable electrode into a further expanded configuration and displacing the catheter tip such that the deformable electrode in its further expanded configuration contacts a portion of the septum while applying electrical RF energy via the deformable electrode to tissue of the septum, thereby ablating tissue of the septum for further widening the opening.

In other words, the final size of the opening forming the interatrial shunt may not be generated in a single ablation step. Instead, the deformable electrode may be deformed in two or more steps into successively further expanded configurations, wherein the cross-sectional area of the deformable electrode is increased from step to step. After each further expanding of the deformable electrode, the electrode may be brought into contact with tissue of the septum and RF energy may be applied for further ablating tissue of the septum. Accordingly, the diameter of the opening in the septum may be increased successively from step to step. Between subsequent steps, pressure measurements may be made for determining the actual LAP and/or RAP. Based on the measured pressure values, it may be determined whether the generated opening has already a sufficient cross-section for acting as an efficient interatrial shunt.

According to an additional embodiment, the catheter comprises a first deformable electrode portion and a second deformable electrode portion, wherein the each deformable electrode portion is configured to be selectively and individually deformed between a retracted configuration and an expanded configuration, wherein, in the expanded configuration, the electrode portion extends along a larger cross-sectional than in the retracted configuration, the cross-sectional area extending perpendicular with regards to a longitudinal direction of the catheter tip, wherein the first deformable electrode portion is arranged at the catheter tip proximally and spaced apart with regards to the second deformable electrode portion. In such embodiment, the method further comprises:
- arranging the catheter tip with the first deformable electrode portion being proximal from the septum and the second deformable electrode portion being distal from the septum, and then
deforming the first and second deformable electrode portions into their expanded configurations to thereby fix the septum between the first and second deformable electrode portions;
- displacing the catheter tip such that at least one of the first and second deformable electrode portions in its expanded configuration contacts a portion of the septum while applying electrical RF energy via the deformable electrode portion to tissue of the septum, thereby ablating tissue of the septum for widening the opening.

Expressed differently, the catheter tip may be provided with two longitudinally separated deformable electrode portions, each of which may be selectively and individually expanded and retracted. Such specific catheter tip may then be arranged within the heart such that the first deformable electrode portion is positioned on one side of the septum whereas the second deformable electrode portion is positioned on the other side of the septum. Upon then expanding each of the first and second deformable electrode portions, the septum is interposed between both electrode portions and thereby fixed relative to the catheter tip.

In such situation, the catheter tip may then be displaced such that the first or the second deformable electrode portion comes into direct mechanical contact with a portion of the septum. Upon simultaneously applying electrical RF energy via the expanded electrode portion, tissue of the septum may be ablated in order to thereby widen the opening in the septum. Due to the preceding fixation of the catheter tip relative to the septum, such widening of the septum opening may be carried out very precisely and/or safe.

According to a further specified additional embodiment, RF energy may be applied to the first deformable electrode while the catheter tip being pushed for displacing the first deformable electrode portion from the right atrium towards the left atrium.

In other words, upon the catheter tip being fixed to the septum as described in the preceding paragraphs, the first deformable electrode portion may be actively pushed from the right atrium towards the left atrium while simultaneously applying RF energy to the first deformable electrode. Thereby, tissue of the septum may be ablated for widening the septum opening. However, instead of applying RF energy to the deformable electrode being arranged in the left atrium and drawing this electrode towards the right atrium, as described further above with regards to other embodiments of the proposed method, RF energy is applied to the deformable electrode portion being arranged in the right atrium while this electrode is pushed towards the left atrium. While such embodiment generally requires that the catheter tip is first fixed relative to the septum before beginning the ablation procedure by applying the RF energy, it may be beneficial to apply the RF energy not to an electrode arranged in the left atrium but to the electrode portion arranged in the right atrium. Particularly, heat locally generated upon applying the RF energy is not mainly induced in the left atrium but in the right atrium. This may be beneficial with regards to a tendency of generating thromboses and/or a dangerousness of any generated thrombosis.

According to a further additional embodiment, a distal end of the catheter tip is configured for puncturing a cardiac septum. The method then additionally comprises puncturing the septum using the distal end of the catheter tip.

Using the for example sharpened or pointed distal end of the catheter tip for generating a transseptal puncture may simplify the surgery procedure as for example no additional puncture needle has to be provided and guided to the septum.

According to a further specified additional embodiment, the catheter further comprises a further electrode arranged at a distal end of the catheter tip and configured for applying electrical RF energy to tissue for locally ablating the tissue. The method then further comprises puncturing the septum by contacting it with the further electrode and applying electrical RF energy via the electrode to tissue of the septum.

Accordingly, the septum may be initially punctured using the further electrode at the distal catheter tip end upon applying RF energy to this electrode, thereby locally ablating septum tissue. Such transseptal puncture using RF ablation may be safer than conventional puncturing using a needle, particularly in cases where for example the fossa ovalis includes cicatrisations. Furthermore, the entire surgery procedure may be simplified.

According to an additional embodiment, the method may further comprise transmitting contrast fluid though the catheter tip while the catheter tip being introduced in one of the right and left atria.

Such transmitting of contrast fluid through for example a channel and an opening at the catheter tip may be helpful upon positioning and/or orienting the catheter tip within the heart, as the contrast fluid may enhance a contrast in an x-ray image used for supervising the cardiac surgery procedure.

According to an additional embodiment, the method further may comprise supplying cooling fluid through the catheter tip to at least a portion of the deformable electrode while electrical RF energy is applied to the deformable electrode.

The cooling fluid may be provided from a reservoir through cooling fluid channels to the catheter tip. There, it may specifically cool a portion of the deformable electrode. For example, a portion of the electrode which is not in direct contact with the septum may be cooled in order to avoid excessive heating of the surrounding blood. The resulting reduced local heat generation may help in preventing risks of thrombogenesis.

Embodiments of the catheter and the method described herein may provide for various advantages. For example, using the proposed method, the generation of an interatrial shunt may be precisely controlled and/or may be realised in a fast manner. Knowledge acquired from other appliances of RF energy ablation may be used upon applying the cardiac surgery method described herein. A control of a success of the applied method, i.e. for example a reduction of the LAP, may be implemented directly together with the surgical procedure, using for example a pressure sensor provided at the catheter tip. Accordingly, the surgeon may for example decide that the opening for the interatrial shunt should be further widened in case the LAP is not yet at a satisfying value. Furthermore, using the distal end of the catheter tip for generating a transseptal puncture may increase a safety of the surgical procedure and/or may simplify the surgical procedure in comparison to using an additional puncture needle.

It shall be noted that possible features and advantages of embodiments of the invention are described herein with respect to various embodiments of a catheter for cardiac surgery, on the one hand, and with respect to various embodiments of a method for cardiac surgery, on the other hand. One skilled in the art will recognize that the features may be suitably transferred from one embodiment to another and features may be modified, adapted, combined and/or replaced, etc. in order to come to further embodiments of the invention.

In the following, advantageous embodiments of the invention will be described with reference to the enclosed drawings. However, neither the drawings nor the description shall be interpreted as limiting the invention.
- Fig. 1: shows a catheter according to a first embodiment of the present invention.
- Fig. 2: shows a catheter according to a second embodiment of the present invention.
- Fig. 3: shows a catheter according to a third embodiment of the present invention.
- Fig. 4A-4I: shows a sequence of steps of a method for cardiac surgery according to an embodiment of the present invention.
- Fig. 5A-5I: shows a sequence of steps of a method for cardiac surgery according to an alternative embodiment of the present invention.

The figures are only schematic and not to scale. Same reference signs refer to same or similar features.

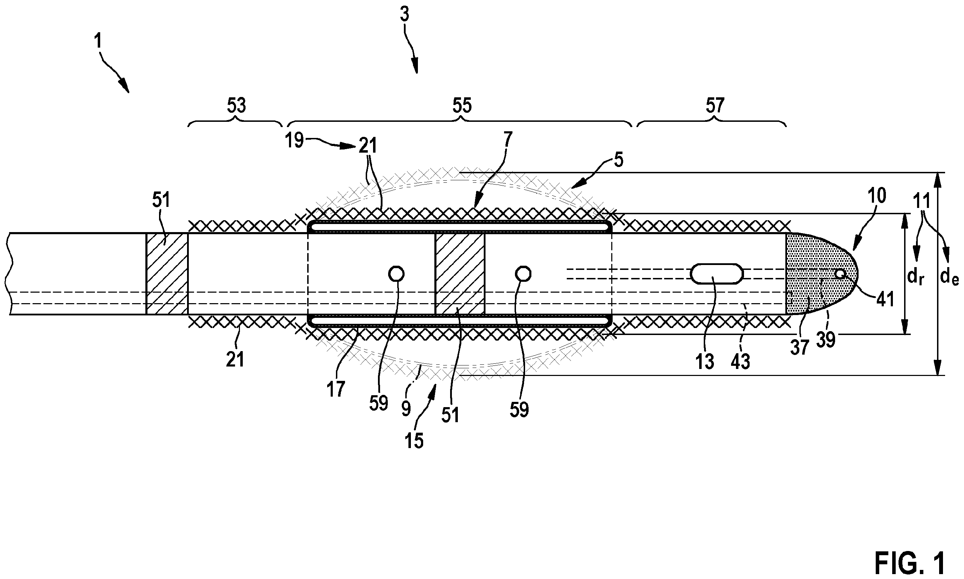

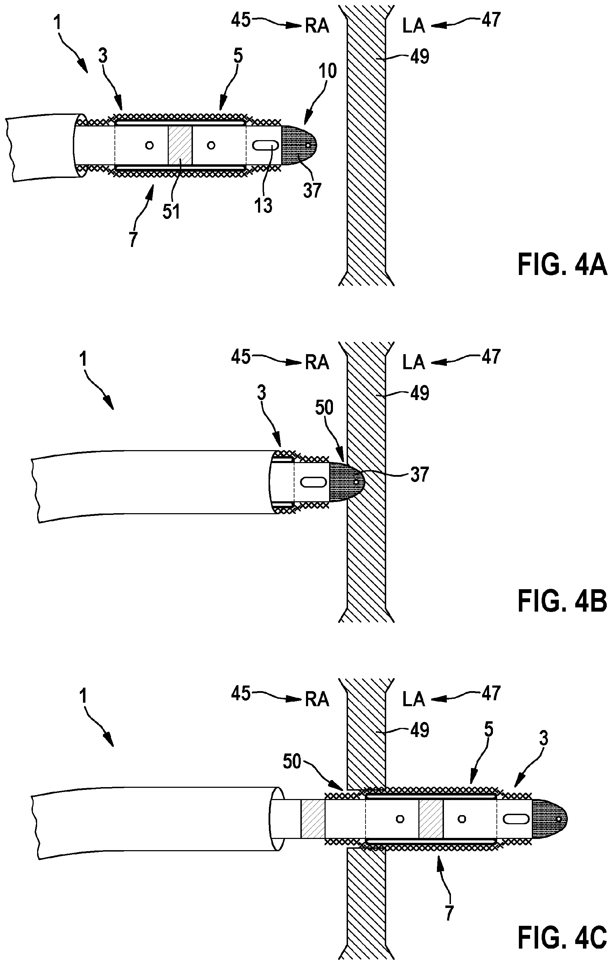

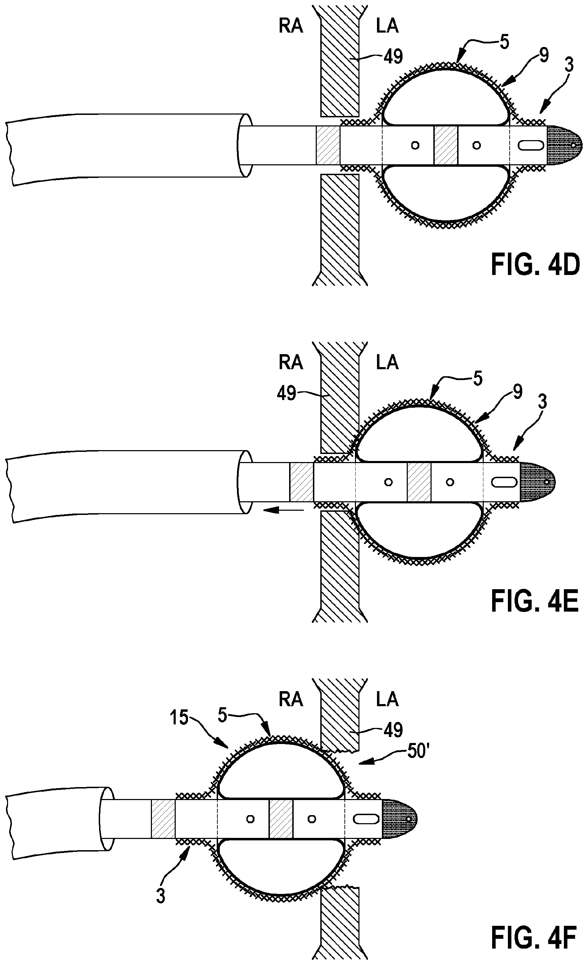

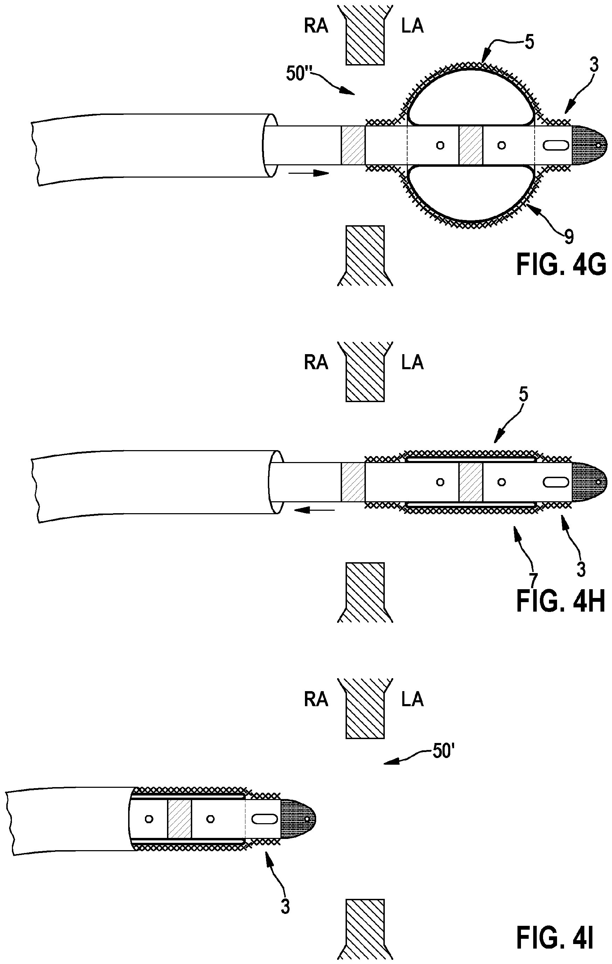

Fig. 1 shows a first example of a catheter 1 for cardiac surgery. The catheter 1 comprises an elongate catheter tip 3 and a deformable electrode 5 arranged at the catheter tip 3. The deformable electrode 5 is configured for applying electrical RF energy to tissue of for example a septum 49 in an atrium (see Fig. 4) in order to create an opening 50" therein serving as an interatrial shunt.

In the example shown in Fig. 1, the deformable electrode 5 comprises an inflatable balloon 17 which is surrounded by an electrically conductive structure 19 formed by a wire mesh 21. The balloon 17 has a tube-like shape and surrounds an outer surface of a catheter body at the catheter tip 3. The balloon 17 may be inflated and deflated by injecting and draining, respectively, fluid via fluid inflation holes 59. The electrically conductive structure 19 is fixed to the catheter tip 3 at a proximal fixation area 53 and at a distal fixation area 57. In an intermediate expandable septal area 55, the electrically conductive structure 19 may be reversibly deformed by inflating or deflating the balloon 17, respectively, between a retracted configuration 7 and an expanded configuration 9 (shown in dotted lines). In the retracted configuration 7, the electrically conductive structure 19 with its expandable septal area 55 is substantially flush with the distal tip 3. In the expanded configuration 9, the expandable septal area 55 radially protrudes from the distal tip 3. Accordingly, in the retracted configuration 7, the deformable electrode 5 has a smaller cross-sectional area 11 with a diameter dᵣ than in the expanded configuration 9 with a diameter dₑ (i.e. dᵣ < dₑ). In the expanded configuration 9, the deformable electrode 9 continuously bulges between the proximal fixation area 53 and the distal fixation area 57. Accordingly, an outermost area 15 in which the expanded deformable electrode 5 has a maximum cross-sectional area 11 is positioned approximately at a longitudinal centre between the proximal and distal fixation areas 53, 57. Therein, the deformable electrode 3 is configured such that a maximum extension of the cross-sectional area 11 in the expanded configuration 9 remains smaller than 12 mm.

The catheter 1 further comprises a pressure sensor 13 configured for sensing a blood pressure in a cardiac chamber. The pressure sensor 13 is arranged at a position distal relative to the deformable electrode 5.

Furthermore, the catheter 1 comprises a distal end 10 of the catheter tip 3. This distal end 10 has a pointed shape. A further electrode 37 is provided at the distal end 10, such further electrode 37 being configured for applying RF energy for ablating tissue contacted by the distal end 10 of the catheter tip 3. Optionally, the further electrode 37 may also be configured for sensing an intracardiac electrogram (IEGM).

Furthermore, a channel opening 41 is provided at the catheter tip 3 near the distal end 10, wherein a contrast fluid may be supplied to the channel opening 41 via a contrast fluid channel 39 (indicated with a dotted line, wherein only an end portion of such contrast fluid channel 39 is visualised in the figure).

Additionally, two x-ray markers 51 are provided at the distal tip 3 at different locations along the longitudinal extension of the distal tip 3.

A cooling fluid channel 43 (only visualised very schematically) may serve for supplying cooling fluid to at least a portion of the deformable electrode 5.

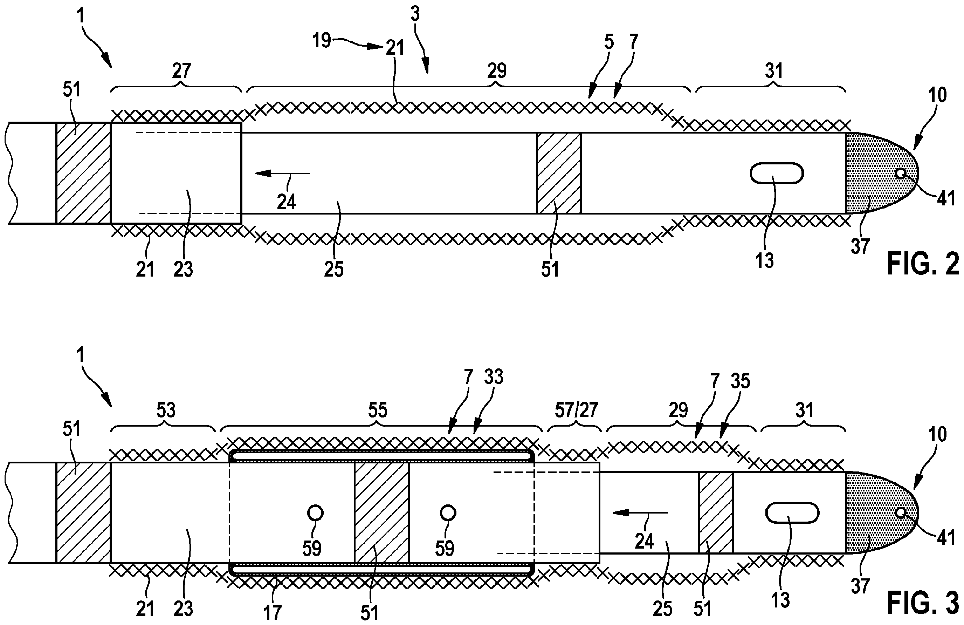

Fig. 2 shows a second example of a catheter 1. Therein, while other features may be embodied in a same or similar manner as described with regards to Fig. 1, the deformable electrode 5 is embodied in a different manner as compared to the embodiment of Fig. 1. Specifically, the catheter tip 3 comprises a first tip portion 23 and a second tip portion 25, which may be displaced relative to each other along a longitudinal motion direction 24. The first and second tip portions 23, 25 may be provided with cylindrical sheaths which are arranged concentrically such as they may be displaced with respect to each other in a telescope-like manner. A proximal end portion 27 of an electrically conductive structure 19 formed by a wire mesh 21 is fixed to the first tip portion 23. A distal end portion 31 of the electrically conductive structure 19 is fixed to the second tip portion 25.

Upon displacing the first and second tip portions 23, 25 along the motion direction 24 relative to each other, a middle portion 29 of the electrically conductive structure 19 may be displaced from a stretched shape to a bulged shape. With the stretched shape, the deformable electrode 5 is in its retracted configuration 7 (as shown in Fig. 2). With the bulged shape, the deformable electrode 5 is in its expanded configuration (not shown in Fig. 2).

Fig. 3 shows a third example of a catheter 1. Therein, the catheter 1 comprises a first deformable electrode portion 33 and a second deformable electrode portion 35. The first deformable electrode portion 33 is configured similarly to the deformable electrode 5 of the embodiment in Fig. 1, whereas the second deformable electrode portion 35 is configured similarly to the deformable electrode 5 of the embodiment in Fig. 2. The first and second deformable electrode portions 33, 35 are arranged at different positions along the longitudinal extension of the catheter tip 3. The first and second deformable electrode portions 33, 35 may be deformed selectively and individually between a retracted configuration 7 (as shown in Fig. 3) and an expanded configuration (not shown in Fig. 3). Therein, an expandable septal area 55 of the first deformable electrode portion 33 is spaced apart in the longitudinal direction from a middle portion 29 of the second deformable electrode portion 35. Accordingly, in their expanded configurations, both deformable electrode portions 33, 35 are spaced from each other by a distance in the longitudinal direction of the catheter tip 3. Such distance is determined by an intermediate region which forms both, the distal fixation area 57 of the first deformable electrode portion 33 as well as the proximal end portion 27 of the second deformable electrode portion 35. Such intermediate region may have dimensions of up to a few millimetres and may roughly corresponds to a thickness of the septum 49.

Fig. 4A-4I visualises a sequence of steps (1) - (9) of a method for cardiac surgery according to a first embodiment.

In a first step (1) shown in Fig. 4A, a catheter 1 is introduced with its catheter tip 3 into a right atrium 45 (indicated in the figure as "RA") of a patient's heart. The deformable electrode 5 is in its retracted configuration 7. The catheter tip 3 may be guided upon its x-ray marker 51 being visualized in an x-ray image.

In a second step (2) shown in Fig. 4B, the catheter 1 is pushed towards the septum 49 such that its further electrode 37 at its distal end 10 contacts the septum 49. Then, electrical RF energy is applied to the further electrode 37, thereby ablating tissue of the septum 49. As a result, the septum 49 is punctured and a small opening 50 is generated in the septum 49.

In a third step (3) shown in Fig. 4C, the catheter tip 3 is then pushed through the opening 50 into the left atrium 47 (indicated in the figure as "LA"). At this stage, the deformable electrode 5 is in its retracted configuration 7. The catheter tip 3 is forwarded until the deformable electrode 5 is positioned within the left atrium 47.

In a fourth step (4) shown in Fig. 4D, the deformable electrode 5 is then expanded to its expanded configuration 9.

In a fifth step (5) shown in Fig. 4E, the catheter tip 3 with its deformable electrode 5 in the expanded configuration 9 is then pulled back towards the septum 49 until the deformable electrode 5 contacts the septum 49. RF energy is applied to the deformable electrode 5 such that tissue of the septum 49 is ablated and the opening 50 is widened.

In a sixth step (6) shown in Fig. 4F, the catheter tip 3 has been pulled back into the right atrium through the widened opening 50'. A diameter of the opening 50' roughly corresponds to a diameter of the expanded deformable electrode 5 at its outermost area 15.

Optionally, in a seventh step (7) shown in Fig. 4G, the catheter tip 3 may again be pushed towards the left atrium. Before such optional step, the deformable electrode 5 may be further expanded such that, upon pushing the deformable electrode 5 through the opening 50' and additionally applying RF energy, further tissue of the septum 49 is ablated and the opening 50"is further widened.

Then, in an eighth step (8) shown in Fig. 4H, the deformable electrode 5 is deflated to its retracted configuration 7.

Finally, in a ninth step (9) shown in Fig. 4I, the catheter tip 3 may be pulled back into the right atrium, before withdrawing the catheter 1 from the patient's heart.

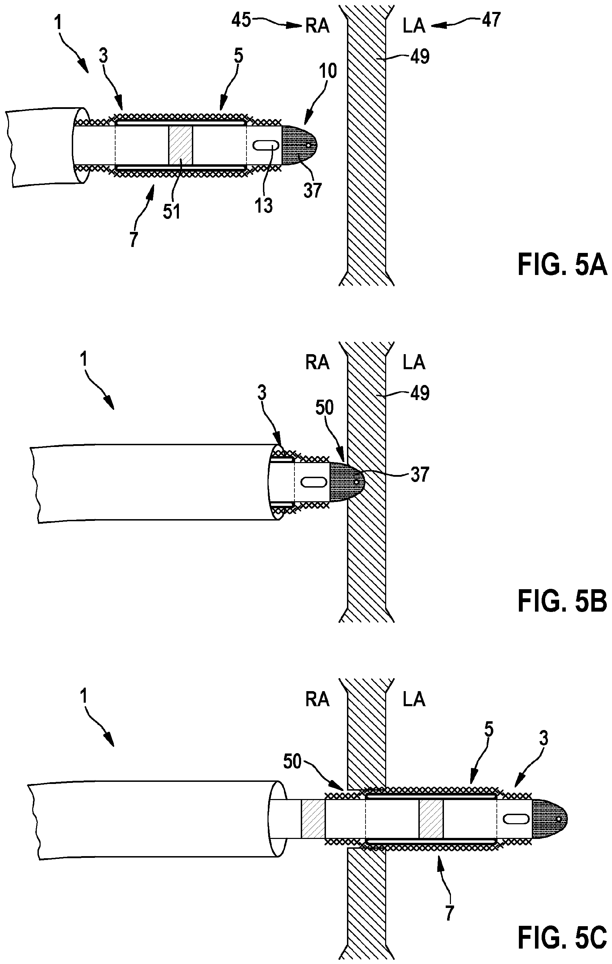

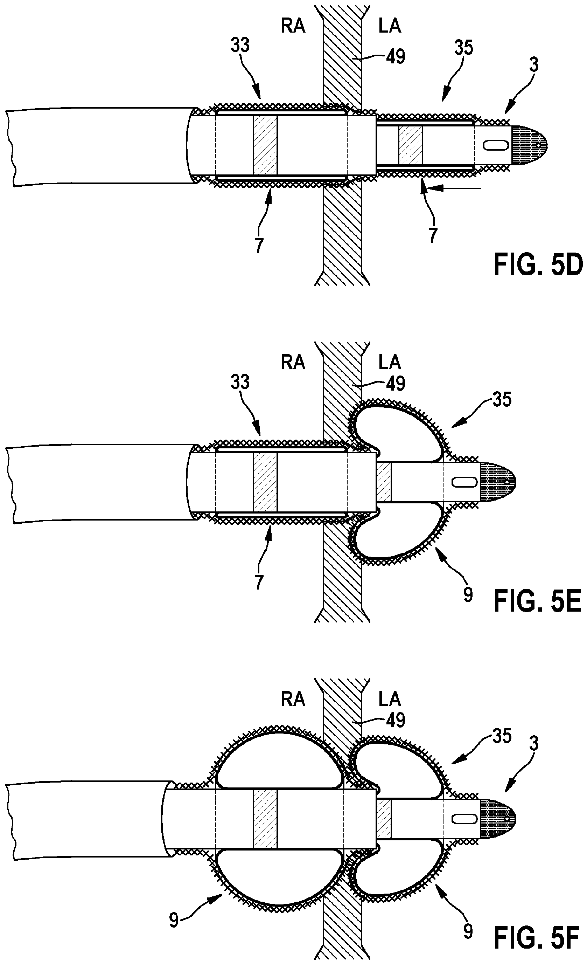

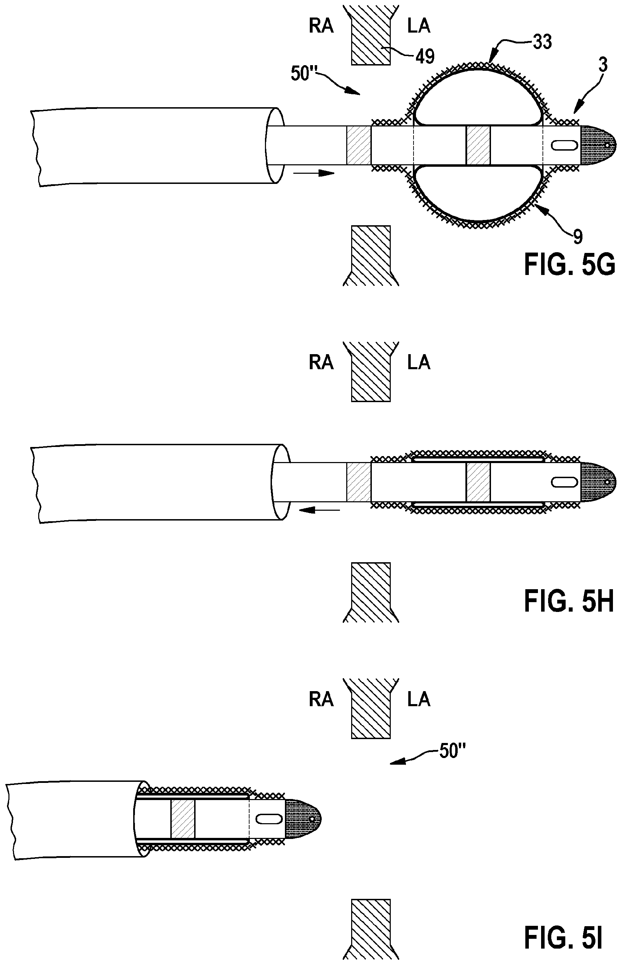

Fig. 5 visualises a sequence of steps (1) - (9) of a method for cardiac surgery according to a second embodiment. Therein, a catheter 1 having the characteristics similar to those described above with reference to Fig. 3 is applied. In such second embodiment, steps (1) - (3) shown in Fig. 5A-5C and (7) - (9) shown in Fig. 5G-5I may be same or similar to the corresponding steps described above for the first embodiment. However, steps (4*) - (6*) shown in Fig. 5D-5F may differ from steps (4) - (6) described above.

Particularly, in step (4^{∗}) shown in Fig. 5D, the catheter tip 3 is arranged such that its first deformable electrode portion 33 is positioned proximal from the septum 49, i.e. in the right atrium, whereas its second deformable electrode portion 35 is positioned distal from the septum 49, i.e. in the left atrium.

In step (5^{∗}) shown in Fig. 5E, the second deformable electrode portion 35 is then expanded to its expanded configuration 9 and the catheter tip 3 is pulled back until the expanded second deformable electrode portion 35 contacts the septum 49.

In step (6^{∗}) shown in Fig. 5F, the first deformable electrode portion 33 is also expanded to its expanded configuration 9. Accordingly, the septum 49 is interposed between the expanded first and second deformable electrode portions 33, 35 such that the catheter tip 3 is fixed with regards to the septum 49. In such fixed configuration, RF energy may then be applied to the first deformable electrode portions 33.

Accordingly, when the catheter tip 3 is subsequently pushed towards the left atrium in step (7), tissue of the septum 49 is locally ablated and the opening 50" is widened.

For controlling a success of the cardiac surgery procedure, the pressure sensor 13 of the catheter 1 may be used to measure blood pressures in at least one of the atria 45, 47. For example, prior to puncturing the septum 49 (e.g. in step (1)), the right atrial pressure may be measured. After transferring the catheter tip 3 to the left atrium (e.g. in step (3)), the left atrial pressure may be measured. Later, after having generated the opening 50" (e.g. in step (6)), the right atrial pressure may again be measured. Optionally, a further measuring of the left atrial pressure may be executed after having retracted the deformable electrode to its retracted configuration (e.g. in step (8)). In case the measured blood pressures are not satisfying, for example an additional widening of the opening 50" may be executed.

Finally, some embodiments of the method for cardiac surgery will be mentioned.

According to an embodiment, the method further comprises:
subsequent to the step of displacing the catheter tip for widening the opening in the septum, expanding the deformable electrode into a further expanded configuration and displacing the catheter tip such that the deformable electrode in its further expanded configuration contacts a portion of the septum while applying electrical RF energy via the deformable electrode to tissue of the septum, thereby ablating tissue of the septum for further widening the opening.

According to an embodiment, the catheter comprises a first deformable electrode portion and a second deformable electrode portion, wherein each deformable electrode portion is configured to be selectively and individually deformed between a retracted configuration and an expanded configuration, wherein, in the expanded configuration, the electrode portion extends along a larger cross-sectional than in the retracted configuration, the cross-sectional area extending perpendicular with regards to a longitudinal direction of the catheter tip,
wherein the first deformable electrode portion is arranged at the catheter tip proximal and spaced apart with regards to the second deformable electrode portion,
the method further comprising:
   - arranging the catheter tip with the first deformable electrode portion being proximal from the septum and the second deformable electrode portion being distal from the septum, and then
deforming the first and second deformable electrode portions into their expanded configurations to thereby fix the septum between the first and second deformable electrode portions;
   - displacing the catheter tip such that at least one of the first and second deformable electrode portions in its expanded configuration contacts a portion of the septum while applying electrical RF energy via the deformable electrode portion to tissue of the septum, thereby ablating tissue of the septum for widening the opening.

According to a further specified version of the preceding embodiment in accordance with the preceding embodiment, RF energy is applied to the first deformable electrode while the catheter tip being pushed for displacing the first deformable electrode from the right atrium towards the left atrium.

According to an embodiment, a distal end of the catheter tip is configured for puncturing a cardiac septum,
wherein the method further comprises: puncturing the septum using the distal end of the catheter tip.

According to an embodiment, the catheter further comprises a further electrode arranged at a distal end of the catheter tip and configured for applying electrical RF energy to tissue for locally ablating the tissue, and
wherein the method further comprises: puncturing the septum by contacting the septum with the further electrode and applying electrical RF energy via the electrode to tissue of the septum.

According to an embodiment, the method further comprises: transmitting contrast fluid though the catheter tip while the catheter tip being introduced in one of the right and left atria.

According to an embodiment, the method further comprises: supplying cooling fluid through the catheter tip to at least a portion of the deformable electrode while electrical RF energy is applied to the deformable electrode.

Finally, it should be noted that the term "comprising" does not exclude other elements or steps and the "a" or "an" does not exclude a plurality. Also elements described in association with different embodiments may be combined. It should also be noted that reference signs in the claims should not be construed as limiting the scope of the claims.

### List of reference numerals

- 1: catheter
- 3: catheter tip
- 5: deformable electrode
- 7: retracted configuration
- 9: expanded configuration
- 10: distal end of the catheter tip
- 11: cross-sectional area
- 13: pressure sensor
- 15: outermost area
- 17: inflatable balloon
- 19: electrically conductive structure
- 21: wire mesh
- 23: first tip portion
- 24: longitudinal motion direction
- 25: second tip portion
- 27: proximal end portion
- 29: middle portion
- 31: distal portion
- 33: first deformable electrode portion
- 35: second deformable electrode portion
- 37: further electrode
- 39: contrast fluid channel
- 41: channel opening
- 43: cooling fluid channel
- 45: right atrium
- 47: left atrium
- 49: septum
- 50: opening in septum
- 51: x-ray marker
- 53: proximal fixation area
- 55: expandable septal area
- 57: distal fixation area
- 59: fluid inflation hole

## Claims

1. Catheter (1) for cardiac surgery, comprising:
an elongate catheter tip (3),
a deformable electrode (5) arranged at the catheter tip (3),
wherein the deformable electrode (5) is configured for applying electrical RF energy to tissue for locally ablating the tissue,
wherein the deformable electrode (5) is configured to be selectively deformed between a retracted configuration (7) and an expanded configuration (9), wherein, in the expanded configuration (9), the deformable electrode (5) extends along a larger cross-sectional area (11) than in the retracted configuration (7), the cross-sectional area (11) extending perpendicular with regards to a longitudinal direction of the catheter tip (3), wherein a maximum extension of the cross-sectional area (11) of the deformable electrode (5) in the expanded configuration is smaller than 9 mm.

2. Catheter according to claim 1, further comprising:
a pressure sensor (13) arranged at the catheter tip (3), the pressure sensor (13) being configured for sensing a blood pressure within a cardiac chamber.

3. Catheter according to claim 2,
wherein the pressure sensor (13) is arranged distally with regards to the deformable electrode (5).

4. Catheter according to one of the preceding claims,
wherein the deformable electrode (5) is configured for applying the electrical RF energy at an outermost area (15) of the deformable electrode (5) being in the expanded configuration.

5. Catheter according to one of the preceding claims,
wherein the deformable electrode (5) comprises an inflatable balloon (17) and an electrically conductive structure (19) at an outer surface of the balloon (17), the conductive structure (19) being deformable together with the balloon (17) upon the balloon (17) being inflated or deflated.

6. Catheter according to claim 5,
wherein the electrically conductive structure (19) comprises one of
- a wire mesh (21) comprising an electrically conductive wire portion extending along a circumference of the balloon (17), and
- an electrically conductive layer structure applied on an outer surface of the balloon (17).

7. Catheter according to one of the preceding claims,
wherein the catheter tip (3) comprises a first tip portion (23) and a second tip portion (25) being longitudinally displaceable with respect to the first tip portion (23),
wherein the deformable electrode (5) comprises a proximal end portion (27), a middle portion (29) and a distal end portion (31), wherein the proximal end portion (27) is fixed at the first tip portion (23) and the distal end portion (31) is fixed at the second tip portion (25).

8. Catheter according to one of the preceding claims,
wherein the catheter (1) comprises a first deformable electrode portion (33) and a second deformable electrode portion (35),
wherein each of the deformable electrode portions (33, 35) is configured to be selectively and individually deformed between a retracted configuration (7) and an expanded configuration (9), wherein, in the expanded configuration (9), the electrode portion (33, 35) extends along a larger cross-sectional area (11) than in the retracted configuration (7), the cross-sectional area (11) extending perpendicular with regards to a longitudinal direction of the catheter tip (3),
wherein the first deformable electrode portion (33) is arranged at the catheter tip (3) proximally and spaced apart with regards to the second deformable electrode portion (35),
wherein, for at least one of the deformable electrode portions (33, 35), a maximum extension of the cross-sectional area (11) of the deformable electrode portion (5) in the expanded configuration (9) is smaller than 9mm.

9. Catheter according to one of the preceding claims,
wherein a distal end (10) of the catheter tip (3) is configured for puncturing a cardiac septum (49), in particular a atrial septum.

10. Catheter according to one of the preceding claims, further comprising:
a further electrode (37) arranged at a distal end (10) of the catheter tip (3) and configured for at least one of
- applying electrical RF energy to tissue for locally ablating the tissue, and
- sensing an intracardiac electrogram.

11. Catheter according to one of the preceding claims, further comprising:
a contrast fluid channel (39) for supplying a contrast fluid and ejecting the contrast fluid at a channel opening (41) at the catheter tip (3).

12. Catheter according to one of the preceding claims, further comprising:
a cooling fluid channel (43) for supplying a cooling fluid to at least a portion of the deformable electrode (5).

13. Method for cardiac surgery, comprising:
- providing a catheter (1), the catheter comprising:
an elongate catheter tip (3), and
a deformable electrode (5) arranged at the catheter tip (3),
wherein the deformable electrode (5) is configured for applying electrical RF energy to tissue for locally ablating the tissue,
wherein the deformable electrode (5) is configured to be selectively deformed between a retracted configuration (7) and an expanded configuration (9), wherein, in the expanded configuration (9), the deformable electrode (5) extends along a larger cross-sectional area (11) than in the retracted configuration (7), the cross-sectional area (11) extending perpendicular with regards to a longitudinal direction of the catheter tip (3),
- introducing the catheter (1) with its catheter tip (3) into a right atrium (45) of the heart;
- optionally puncturing a septum (49) separating the right atrium (45) from a left atrium (47) of the heart thereby generating an opening (50) in the septum (49) and transferring the catheter tip (3), with the deformable electrode (5) being in the retracted configuration (7), through the generated opening (50) or an already existing opening (50) into the left atrium (47) such that at least a portion of the deformable electrode (5) is positioned within the left atrium (47),
- deforming the deformable electrode (5) into its expanded configuration (9);
- displacing the catheter tip (3) such that the deformable electrode (5) in its expanded configuration (9) contacts a portion of the septum (49) while applying electrical RF energy via the deformable electrode (5) to tissue of the septum (49), thereby ablating tissue of the septum (49) for widening the opening (50").

14. Method according to claim 13, further comprising:
measuring a pressure within the left atrium (45) and, optionally, determining a degree of expansion into which the deformable electrode (5) is deformed based on the measured pressure in the right atrium (45).

15. Method according to one of claims 13 and 14, further comprising:
measuring a pressure within the right atrium (47) and, optionally, determining subsequent processing steps based on the measured pressure in the left atrium (47).
